# EUROPEAN PATENT SPECIFICATION

(11) **EP 2 207 511 B1**
(45) Date of publication and mention of the grant of the patent: **14.08.2019**
(21) Application number: 07822246.0
(22) Date of filing: 06.11.2007
(51) Int. Cl.: A61F 13/15

(54) **METHOD OF MANUFACTURING A BELT OR A SIDE PANEL OF AN ABSORBENT ARTICLE, LAMINATE AND ABSORBING ARTICLE**
VERFAHREN ZUR HERSTELLUNG EINES GURTS ODER EINES SEITENTEILS EINES SAUGFÄHIGEN ARTIKELS, LAMINAT UND SAUGFÄHIGER ARTIKEL
PROCÉDÉ DE FABRICATION D'UNE COURROIE OU D'UN PANNEAU LATÉRAL D'UN ARTICLE ABSORBANT, STRATIFIÉ ET ARTICLE ABSORBANT

(43) Date of publication of application: 21.07.2010
(73) Proprietor: Essity Hygiene and Health Aktiebolag, 405 03 Göteborg (SE)
(72) Inventor: BÄCK, Lucas, 405 05 Göteborg (SE); DAHLQVIST, Conny, 405 05 Göteborg (SE)
(74) Representative: Hoffmann Eitle
(86) International application number: PCT/EP2007/061924
(87) International publication number: WO 2009/059634

(56) References cited:
- US-A- 5 540 796
- US-A- 5 876 531
- US-A1- 2002 193 776

## Description

### 1. TECHNICAL FIELD

The present invention relates to the field of absorbent articles, in particular to baby diapers or adult incontinence products. More specifically, the invention relates to belts or side panels of these absorbent articles, which serve to fasten the diapers or incontinence products to a wearer.

Absorbent articles of this kind usually comprise an absorbent structure including a back sheet, a top sheet and an absorbent core situated there between. The top sheet is intended to be directed towards the user when the absorbent article is worn. To fasten the absorbent articles around the waist of a wearer, a fastening system is provided on the absorbent structure, typically in the form of a belt system or in the form of side panels. Conventionally, the belts or side panels extend laterally from the absorbent structure such that they can be connected to one another, or to a landing zone on the absorbent structure, in order to fasten the absorbent article to the wearer.

It is desired that the belts or side panels of the absorbent articles provide, on the one hand, a comfortable fit to the wearer, in particular when it comes to wearing adult incontinence products, in particular by bedridden people, but, on the other hand, provide a secure and tight fit to accommodate for different activity levels of the wearer. Accordingly, different concepts of providing the belts or side panels for absorbent articles have been proposed.

### 2. BACKGROUND OF THE INVENTION

Absorbent articles, in particular adult incontinence products, are worn by the respective wearer typically over a considerable period and on a day-to-day basis. Accordingly, a comfortable fit of the absorbent article is crucial in order to increase the well-being of the wearer. In the case of adult incontinence products, absorbent articles that are worn around the lower part of the trunk are usually used by bedridden people, in particular elderly people. Depending on their mental state and activity level, different types of incontinence products are usually used to accommodate for the wearer's specific needs. Incontinence products that are applied to bedridden people are usually applied to the wearer by nursing staff or other caretakers.

Accordingly, a balance has to be found between a tight fit of the incontinence product in order to reduce the occurrence of leaks or shifting of the absorbent article due to movements of the wearer, the actual wearing comfort in order to increase the well-being of the wearer of the product, and a good applicability to facilitate convenient, quick and trouble-free application of the incontinence product, in particular by nursing staff or other caretakers. The belts or side panels play an important roll in this respect as they are the elements actually actuated and/or manipulated by nursing staff or caretakers. Accordingly, it is important to improve reliability and manufacturing efficiency and product quality in this respect.

In order to accommodate for the different positions of a wearer during the day-to-day use of incontinence products, JP 09-154884 A proposes a disposable diaper having, included in a side panel, an expandable part at the base ends of the respective side panels. The expandable part serves to accommodate different wearing positions in order to increase the tight fit of the diaper.

EP 1 269 949 A2 relates to a method of making a winged absorbent article. According to this method, a web of wing- making material is fed together with the chassis of the absorbent article and folded wing portions are severed from the web of wing-making material and are attached to the chassis .

EP 1 004 285 A1 relates to a process for applying discrete web portions to a receiving web. According to this disclosure, a web is fed into a web transfer apparatus at a certain speed, and the web is then cut into web portions and applied to a receiving web.

US 5,540,796 describes a technique for forming an elasticized article including the steps of providing first and second webs of elasticized side panel material. The side panel material is constructed to be elastically stretchable at least along an appointed lateral cross direction. At least one first fastener is attached to the first web of side panel material, and at least one second fastener is attached to the second web of side panel material. A web of bridge material is provided with first and second side edge regions thereof. The first web of side panel material is attached to the first side edge region of the web of bridge material, and the second web of side panel material is attached to the second side edge region of the web of bridge material. The second web of side panel material is arranged to provide a cross-directional alignment between at least one corresponding, laterally opposed pair of the first and second fasteners. The web of bridge material and the first and second webs of side panel material are divided to provide at least one composite bridge assembly having a bridge member interconnecting a laterally opposed pair of first and second side panel members. The composite bridge assembly is secured to an appointed article web with the first and second side panels of the laterally opposed pair of side panel members located at opposite side regions of the article web.

US 5,876,531 describes a method for forming a plurality of fastener components comprising providing a composite web which includes a web of hook material. The hook material includes a hook base layer which has a longitudinally extending medial section located between first and second, laterally opposed, longitudinally extending side sections of the web of hook material. Each of the side sections has a plurality of hook elements which are integrally formed with the base layer and extend away from a base plane of the hook base layer, and the hook elements are configured to operably engage a selected, cooperating loop material to provide an operative fastening. The medial section has a relatively lower density of the hook elements per unit area, as compared to the side sections. The web of hook material has an extending section of carrier web material attached to extend laterally outboard from each of the side sections of the web of hook material. Each section of carrier web material may have an extending web of panel material attached to extend laterally outboard from each section of carrier web material. At least the web of hook material is divided along a serpentine division line which extends generally longitudinally along the web of hook material, and the composite web is selectively segmented to provide for the plurality of fastener components.

US 2002 193776 discloses an absorbent article having a belt comprising first and second belt halves. The belt halves are attached to or adapted to be attached to the back portion of the article and to the front portion of the article in such a way that the article assumes a pant-like shape.

### 3. SUMMARY OF THE DISCLOSURE

It is an object of the present invention to provide a method of manufacturing a belt or a side panel of an absorbent article which improves the efficiency of the manufacture as well as the quality of the resulting belt or side panel as well as of the resulting absorbent article. It is a further object of the present invention to provide an absorbent article with such a belt or side panel with improved quality and application characteristics.

These objects are achieved by a method of manufacturing a belt or a side panel according to claim 1 as well as by an absorbent article according to claim 11.

In this disclosure, the term "belt" is understood to mean an elongate structure that is used to fasten the absorbent article to a wearer. The belt is situated to be joined together around the waist of the wearer. Typically, the belt is fixedly attached to the absorbent article in a front portion or a rear portion and can be attached to the respective other of the front and rear portion of the absorbent article in order to fasten the absorbent article to the wearer. In order to attach the belt to the absorbent article, the rear part, or alternatively the front part, of the belt exhibits fixing devices that can be attached to the absorbent article or, in an alternative, to another belt. In the arrangement of the absorbent article fastened to the wearer, the belts form a part of the waist part of absorbent article. When putting on a belt diaper, the belt is fixed, in a first stage, around the waist of the wearer. The free transverse edge of the absorbent part of the diaper is then passed between the wearer's legs and is attached to the side of the belt facing away from the wearer. An example of an absorbent article in the form of a belted diaper, i.e. a diaper having a belt as a fastening structure, is shown in WO 2006/065177 A1.

In this disclosure, the term "side panel" is understood to relate to a part of an absorbent article that is situated on the side of the absorbent article when the absorbent article is worn, constituting a substantial portion of a pants-like absorbent article. Typically, side panels are re-sealable such that the pants-like absorbent article can either be put on or taken off in the same manner as regular pants, or, in an alternative, can be put on or taken off in the manner as conventional diapers. The side panel is typically directly attached to the absorbent portion of the absorbent article. Examples of diapers with side panels are shown in WO 03/082168 A1 or WO 03/094815 A1.

In other words, belts and side panels differ, *inter alia*, with respect to their dimensions. Belts have a rather elongate and usually rectangular configuration and have a length of typically half the circumference of the waist of the target wearer. Side panels have a somewhat complex shape, accommodating sections of a leg hole along one of their edges and constituting a waist portion on another edge. The length of the side panels in the circumferential direction extends typically only between front part and rear part of the footprint of a top sheet and/or bottom sheet of the absorbent article and typically extends around one fourth of the circumference of the waist of the wearer. In the lateral direction, belts typically have a considerably smaller dimension than side panels.

Accordingly, the method of manufacturing a belt or a side panel of an absorbent article comprises the steps of feeding a first web of material and a second web of material in the machine direction; cutting the first web of material along a cut line extending along the machine direction into at least a first web portion and a second web portion, cutting the second web of material along the machine direction and along a cut line into at least a third web portion and a fourth web portion providing a first elastic web material and a second elastic web material and feeding the elastic web materials in the machine direction; producing a first laminate by attaching the first elastic web material to the first web portion and the second web portion of the first web of material so as to bridge the cut line in order to provide for elasticity in the resulting laminate; producing a second laminate by attaching the second elastic web material to the third web portion and the fourth web portion of the second web of material, so as to bridge the cut line in order to provide for elasticity in the resulting laminate; and cutting two individual belts or side panels from the laminates along the cross machine direction, wherein the first and second laminate each have an attachment side for attaching the belts or side panels to a carrier web, and wherein the two laminates are registered with one another before being cut such that the attachment side of the second laminate extends slightly in the lateral direction over the lateral edge of the first laminate; and simultaneously cutting two individual belts or side panels from the registered laminates.
By the provision of a single web of material in the machine direction only, which is also the web of material that provides the main section of the belt or the side panel, and the subsequent cutting step of the web of material along the machine direction, a very precise feeding and manufacturing of the belt or side panel is enabled by the method. In particular, as the web of material has a constant width, the resulting belt or side panel also has a constant width, and the quality of the sections of the belt, made up from the web of material, have a constant quality, as they are made up from the same material.

As a result of the step of producing a laminate by reconnecting the two previously cut first and second web portions, the resulting laminate receives a very precise layout. In particular, as the first web portion and the second web portion stem from a single web of material, they can easily be aligned in a parallel manner. Specifically, the web handling is improved as only a single web of material has to be fed and aligned in the machine.

A first lateral side section of the elastic web material is joined to the first web portion and the second lateral side section of the elastic web material is joined to the second web portion.

It is advantageous displacing the first web portion and the second web portion with respect to one another such that a gap is provided between the first web portion and the second web portion before the elastic web material is attached to produce the laminate, and the elastic web material is attached such that it bridges the gap. By the provision of such a gap, the elastic properties of the resulting belt or side panel can be precisely adjusted.

Nevertheless, in a further advantageous version of the method, the first web portion and the second web portion are held in close relationship with respect to one another, and the attachment positions of the first lateral side section and the second lateral side section on the first and second web portions are placed at a distance from the actual cut line. As the attachment positions, in which the elastic web material is actually attached to the first web portion and to the second web portion, are spaced apart from the cut line, a considerable section of the elastic web material can act elastically between the first web portion and the second web portion.

In a preferred embodiment, the elastic web material is elastic in the cross machine direction. Furthermore, in some applications, it can be advantageous that the elastic web material is essentially non-elastic in the machine direction, in particular such that it has the same elasticity as the web of material in the machine direction.

Preferably, in a further step, wing tabs are attached to one lateral side of the laminate at predetermined intervals . The dimensions of the predetermined intervals preferably correspond to the dimensions of the individual belt portions in the machine direction, such that at least one wing tab is situated on each individual belt.

In a preferred variant of the method, the elastic web material is fed in the form of a longitudinal strip of a cross machine dimension which is larger than the cross machine dimension of the gap. In particular, the cross machine dimension of the elastic web material is preferably smaller than the cross machine dimension of the web of material before it is cut.

It is preferred in the method that the first web portion and the second web portion, on the one hand, and the third web portion and the fourth web portion, on the other hand, are spaced apart from one another by the same amount, resulting in an equally spaced gap between the first web of material and the second web of material .

Furthermore, in a preferred variant, an elastic web material is fed in the machine direction and the elastic web material is cut along the machine direction into a first elastic web portion and a second elastic web portion. The two laminates are registered with one another before being cut and two individual belts or side panels are cut simultaneously from the registered laminates.

Preferably, in the method, an absorbent structure, in particular including a back sheet, a top sheet and an absorbent core, is fed in the machine direction of the laminate and the individual belt portion or the individual side panel portion is attached to the absorbent structure.

The attachment of the individual belt structure or the individual side panel is preferably effected while travelling in the same direction as the absorbent structure such that they are moving at the same speed in the same direction.

Preferably, in order to increase the wearing comfort, the individual belt or the side panel are fixedly attached to the absorbent structure only on one of the lateral sides of the individual belt or the side panel. This enables using the belt or the side panel to be folded around a wearer. However, the other lateral side may be attached to the absorbent structure by a releasable bond in order to increase the production efficiency and the packaging efficiency.

It is preferred in accordance with another aspect of the invention that an absorbent article is provided, comprising at least two belts or side panels made in accordance with the method described herein.

### 4. BRIEF DESCRIPTION QF THE DRAWINGS

Various exemplary embodiments of the method will now be described in greater detail, by way of example only, with reference to the attached drawings, in which:

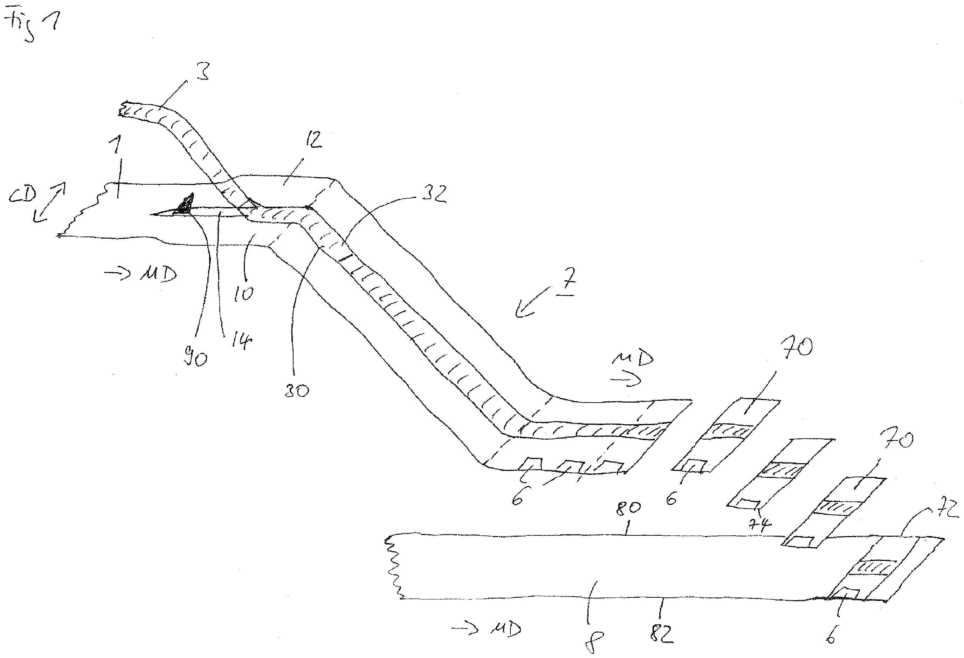

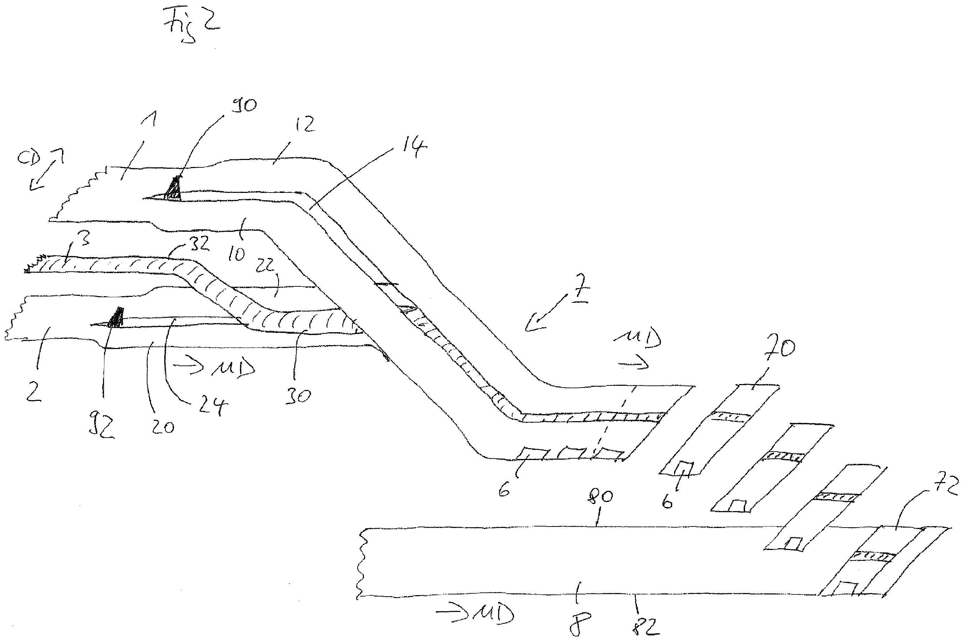

Figure 1 is a schematic perspective view of an exemplary method of manufacturing a belt or side panel of an absorbent article, providing one web of material and one elastic web material; Figure 2 is a schematic perspective view of another exemplary method showing two webs of material between which a single elastic web material is sandwiched and joined to the two webs of material;

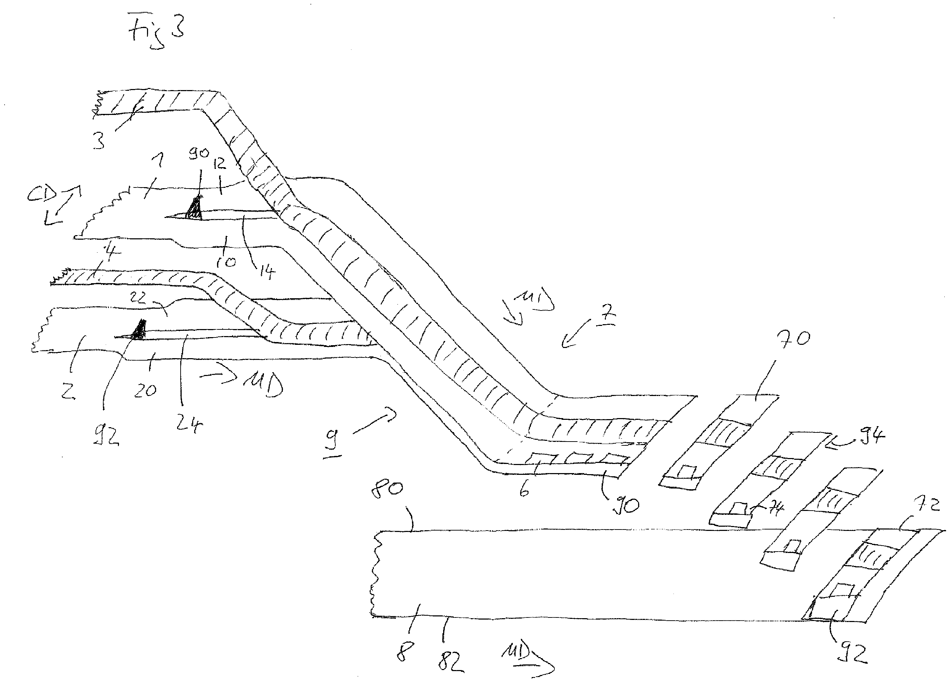

Figure 3 is a schematic perspective view of a method of manufacturing two belts or side panels in accordance with the invention, using two webs of material and two elastic web materials, wherein one elastic web material is joined to the first web of material and a second elastic web material is joined to the second web of material;

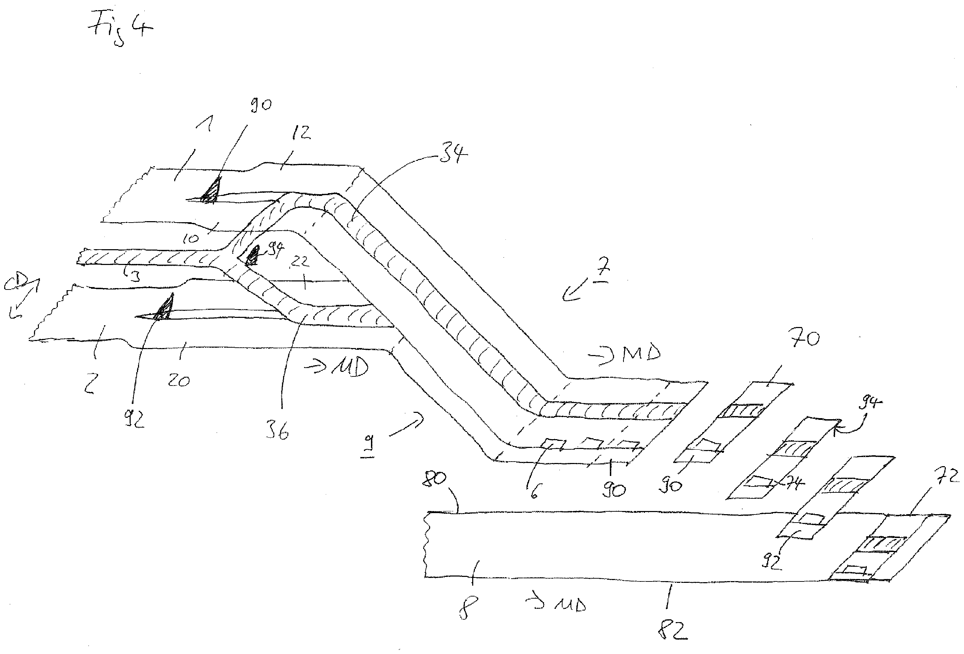

Figure 4 is a schematic perspective view of another method for manufacturing two belts of side panels in accordance with the invention, using two webs of material and a single elastic web material, wherein the single elastic web material is cut into a first elastic web portion and a second elastic web portion, wherein the first elastic web portion is joined to the second web of material, and the second elastic web portion is joined to the first web of material;

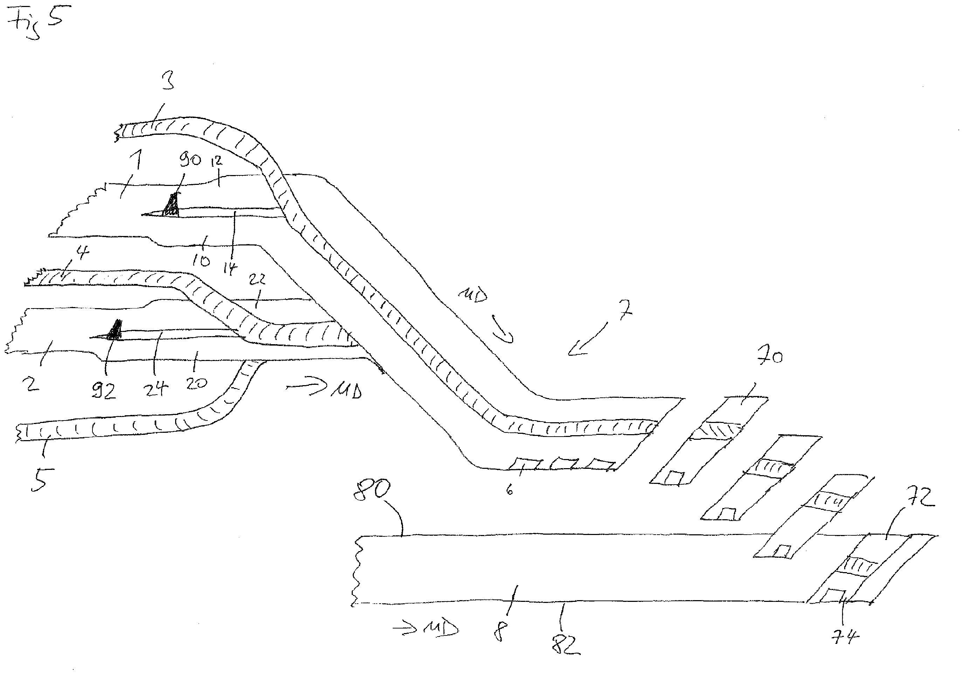

Figure 5 is a schematic perspective view of yet another method of manufacturing a belt or a side panel in accordance with the invention, using a first and a second web of material and three elastic web materials, wherein one elastic web material is joined to the first web of material and is substantially sandwiched between the first and the second webs of material and the two other elastic web materials are joined to the respective outer sides of the first and second webs of material;

### 5. DETAILED DESCRIPTION OF PREFERRED EMBODIMENTS QF THE DISCLOSURE

Preferred embodiments of the method of manufacturing of a belt or a side panel of an absorbent article will now be described in detail with reference to the drawings, wherein similar reference numerals identify similar or identical elements and repeated description of these features in the respective embodiments is omitted.

Figure 1 shows a schematic perspective view of a manufacturing method. A first web of material 1 is provided and is fed in the machine direction MD towards a cutting means 90, which is schematically shown in the Figure as a knife. The web of material 1 is cut by the cutting means 90 into a first web portion 10 and a second web portion 12. A gap 14 between the first and second portions 10, 12 is present, resulting from a slight displacement of the first web portion 10 and the second web portion 12 in the cross machine direction CD with respect to one another.

Accordingly, after having passed the cutting and displacement step, the first web portion 10 and the second weh portion 12 are carried parallel to one another in the machine direction MD, and a gap 14 is present between the first web portion 10 and the second web portion 12.

It should be understood, however, that even though the cut is situated in the embodiment shown basically in the middle of the web of material 1, it could also be situated in any other suitable position across the width of the web of material . In particular, the cut could be situated, for example, in a position of 1/3, 1/4, 1/5 or 1/6 of the width of the web of material, or in any other suitable position.

An elastic web material 3 is provided which joins the first web portion 10 and the second web portion 12. The elastic web material 3 basically bridges the cut, or the gap 14, between the first web portion 10 and the second web portion 12.

In other words, the elastic web material 3 is joined to its first lateral side section 30 to the first web portion 10 of the web of material 1 and with its second lateral side section 32 to the second web portion 12 of the web of material 1. In this respect, it is to be understood that the position of the actual joint between the elastic web material 3 and the first web portion 10 and the second web portion 12 might be in any suitable position on the web of material. Preferably, the joints are not necessarily situated on the lateral edges of the elastic web material, but rather towards the middle of the elastic web material to ensure a secure fastening. Accordingly, the elastic web material 3 bridges the gap 14 between the first web portion 10 and the second web portion 12 and, thus, reconnects the two previously cut-apart web portions 10, 12. In the method shown, at least the section of the elastic web material 3, which bridges the gap 14, acts as an elastic element which provides for elastic movement in the cross-direction CD in the resulting laminate 7. To be more specific, the section of the elastic web material 3 extending between the positions of the joints of the elastic web material with the first and second web portions 10, 12 serves as the elastic means. Accordingly, the laminate 7 comprises the originally fed web of material 1, cut into a first web portion 10 and a second web portion 12, and re-connected by the elastic web material 3.

The handling of the laminate 7 and the manufacturing method are improved if the elastic web material 3 is only elastic in the cross machine direction CD, but is essentially non-elastic in the machine direction MD. This is due to the fact that, in an arrangement in which the elastic web material 3 is essentially non-elastic in the machine direction MD, or, more preferably, has an elastic module in the machine direction which is identical to that of the web of material 1 in the machine direction, any wrinkling due to an elastic tension of the elastic web material 3 in the machine direction MD can be avoided. Nevertheless, if the elastic web material 3 is elastic in the cross machine direction CD, an elastic effect can be seen in the cross machine direction CD of the laminate 7.

In a preferred manufacturing step, wing tabs 6 are attached to the laminate, in particular to the outer lateral side of the first web portion 10, wherein the wing tabs 6 are spaced at intervals with respect to one another which essentially correspond to the MD dimensions of the individual belts 70 which are cut from the laminate 7. In particular, more than one wing tab can be situated on each individual belt, leading to a different pitch of the wing tabs.

In a subsequent step, the laminate 7 is cut in the cross machine direction CD into individual belts 70 or side panels. In particular, the individual cuts are carried out in the sections of the laminate 7 between the wing tabs 6 such that the wing tabs remain intact.

In a preferred step, an absorbent structure 8, which is shown in Figure 1 schematically in the form of a carrier web 8, is fed in the same direction as the laminate 7. Accordingly, the laminate 7 and the carrier web 8 travel in the same direction. The carrying speeds of laminate 7 and carrier web 8 are distinct, but the speeds of the belt 70 and the carrier web 8 are preferably identical in the section in which the belts 70 are joined to the carrier web 8. The belt 70 is then joined to the carrier web 8 on its attachment side 72, wherein the attachment side 72 is situated opposite the side 74 of the belt 70 onto which the wing tabs 6 were placed.

This enables an efficient and continuous manufacture of absorbent articles. As belts are only situated in discrete portions of the carrier web, the speed of the laminate 7 is considerably lower than the speed of the carrier web 8.

The process of bonding the belt 70 to the carrier web 8 may be carried out according to different methods. According to one preferred method, the carrier web 8 is the top sheet (or the back sheet) of the absorbent article thus manufactured. The belt 70 is placed in an overlapping, or partially overlapping, relationship onto the carrier web 8 and the attachment side 72 of the belt 70 is joined to the carrier web 8, e.g. by applying an adhesive or by heat bonding. In a subsequent step, the back sheet (or top sheet) is applied to the top sheet (or back sheet), sandwiching and joining the attachment side 72 of the belt 70 between them.

In another preferred method of attaching the belt 70 to the carrier web 8, the belt 70 may be attached to the carrier web 8 by bending or folding the side 72 around one the edges 80 of the carrier web 8, resulting in a situation in which the attachment side 72 of the belt 70 is situated on the opposite surface of the carrier web 8 than the remainder of the belt 70. This method is described e.g. in WO 99/37263 A1.

Both methods of bonding the belt 70 to the carrier web 8 can also be used in all following methods described below.

Returning to Figure 1, even though a gap 14 is shown between the first web portion 10 and the second web portion 12, it is envisaged that in an alternative embodiment the first web portion 10 and the second web portion 12 are held in a close relationship with respect to one another such that only the cut itself is present at the position where the gap 14 is shown in Figure 1. The attachment positions of the first lateral side section and the second lateral side section on the first and second web portions, however, are placed at a distance from the actual cut line. In such an embodiment, the effective elastic portion of the elastic web material can be achieved by placing the points of attachment of the first lateral side section 30 and the second lateral side section 32 at a distance from the actual cut line. In other words, a sufficient section of elastic material is provided between the positions in which the elastic web material is bonded to the web of material, in order to provide for elasticity in the resulting laminate.

Figure 2 shows a schematic perspective view of another method of manufacturing a belt or a side panel of an absorbent article. A first web of material 1 is fed towards a cutting means 90, cutting the web of material 1 into a first web portion 10 and a second web portion 12, providing, by displacement in the cross machine direction CD, a gap 14 between the first web portion 10 and the second web portion 12.

A second web of material 2 is also fed in the machine direction MD towards a second cutting device 92, cutting the second web of material 2 into a third web portion 20 and a fourth web portion 22, providing, by displacement in the cross machine direction CD, a gap 24 between the third web portion 20 and the fourth web portion 22. The width of the gaps 14, 24 in the cross machine direction CD are kept essentially at the same value.

An elastic web material 3 is fed between the first web of material 1 and the second web of material 2 and is joined to the first and second web portions 10, 12 such that its first lateral side section 30 is joined to the third web portion 20 as well as with the first web portion 10. The second lateral side section of the elastic web material 3 is joined to the fourth web portion 22 and to the second web portion 12.

The resulting laminate 7 sandwiches the elastic web material 3 between the first web material 1 and the second web of material 2 and the elastic web material 3 is joined to both layers of web material 1, 2.

The method of attachment of the finally cut individual belts 70 to the carrier web 8 corresponds to that shown and described with respect to Figure 1.

Figure 3 shows the present method of manufacturing two belts or side panels of an absorbent article simultaneously. The basic structure resembles that shown in and explained with respect to Figure 2. Nevertheless, in addition to the first elastic web material 3, a second elastic web material 4 is provided and fed in the machine direction MD. As can be seen in Figure 3, the second elastic web material 4 is placed on the second web of material 2, thus bridging the gap 24 between the third web portion 20 and the fourth web portion 22 and joining the third and fourth web portions 20, 22. The first elastic web material 3 and joins the first and second web portions 10, 12.

As a result, a first laminate 7 and a second laminate 9 having an elastic portion, provided by the first and second elastic web materials 3, 4 are formed. The two laminates 7, 9 are not attached to one another but are in a fixed positional relationship with respect to one another, i.e. they are registered with one another. In particular, the attachment side 92 of the second laminate 9 extends slightly in the lateral direction over the lateral edge of the first laminate 7. After been cut, this results in a first belt 70 stemming from the first laminate 7 made of the first web of material 1 and the first elastic web material 3, and a second belt 90 stemming from the second laminate made of the second web of material 2 and the second elastic web material 4. The belts 70, 90 are joined at attachment sides 72, 92 to the carrier web 8. The attachment sides 72, 92 are situated opposite to one another, resulting in a structure of a carrier web 8 with two belts 70, 90 attached to it, wherein the belts are joined to two opposite edges 80, 82 of the carrier web 8 such that the non-joined sides 74, 94 of the belts 70, 90 are free, or attached to one another by a releasable bond, to be extended around the hip of a wearer.

In other words, unlike in the method shown in Figure 2, the two laminates 7, 9 are not attached to one another but result in two separate belts 70, 90 which can be attached to the carrier web 8 in a fixed positional relationship (i.e. in a registered manner).

Nevertheless, in an alternative arrangement the first web of material 1 and the second web of material 2 may be joined together, resulting in a laminate in which the elastic web material 3 and the second elastic web material 4 are placed, in the section of the gap 14, 24, on top of one another. Thus, the gap 14 between the first web portion 10 and the second web portion 12 as well as the gap 24 between the third web portion 20 and the fourth web portion 22 are bridged by the second elastic web material 4. In addition to that, the gap 14 between the first web portion 10 and the second web portion 12 is bridged by the first elastic web material 3. This method results in a laminate 7 which has a high strength.

Figure 4 shows a perspective view of a further preferred method of manufacturing belts or side panels of an absorbent article. The two laminates 7 and 9 manufactured by this method essentially resemble those described with reference to the method described with respect to Figure 3. Nevertheless, in the method described, only a single elastic web material 3 is fed in the machine direction and is cut into a first elastic web portion 34 and a second elastic web portion 36. The elastic web material 3 is cut by a cutting means 94 which is schematically shown, by way of example only, as a knife.

The first elastic web portion 34 joins the first and second web portions 10, 12, substantially in the same manner as the first elastic web material 3 in Figure 3, and the second elastic web portion 36 joins the third and fourth web portions 20, 22, substantially in the same manner as the second elastic web material 4 in Figure 3.

In the embodiment shown in Figure 4, the second web of material 2 is shifted slightly in the cross machine direction CD with respect to the first web of material 1. Accordingly, the second laminate 9, in particular, the third web portion 20 and, thus, the attachment side 92, extends beyond the non-joined side 74 of the first laminate 7. The resulting belts 70, 90, however, have the same dimensions (length) in the lateral (cross machine} direction.

Figure 5 shows yet another schematic perspective view of still another preferred method. In the method, a third elastic web material 5 is fed in the machine direction MD and is joined to the lower surface of the second web of material 2. The first elastic web material 3 is joined to the upper surface of the first web of material 1 and the third elastic web material 5 is joined to the lower surface of the second web of material 2. Additionally, a second elastic web material 4 is sandwiched between the first web of material 1 and the second web of material 2 and joined to both webs of material 1 and 2. The resulting laminate 7 has, accordingly, three layers of elastic web material and two layers of webs of material attached on top of one another, leading to a relatively strong laminate.

## Claims

1. Method of manufacturing at least two belts (70) or side panels of an absorbent article, the method comprising the steps of feeding a first web of material (1) and a second web of material (2) in the machine direction (MD);
cutting the first web of material (1) along a cut line extending along the machine direction into at least a first web portion (10) and a second web portion (12), cutting the second web of material (2) along the machine direction and along a cut line into at least a third web portion (20) and a fourth web portion (22);
providing a first elastic web material and a second elastic web material and feeding the elastic web materials (3, 4) in the machine direction;
producing a first laminate (7) by attaching the first elastic web material to the first web portion and the second web portion of the first web of material so as to bridge the cut line in order to provide for elasticity in the resulting laminate;
producing a second laminate (9) by attaching the second elastic web material to the third web portion and the fourth web portion of the second web of material, so as to bridge the cut line in order to provide for elasticity in the resulting laminate; and
cutting two individual belts (70, 90) or side panels from the laminates along the cross machine direction (CD),
wherein the first and second laminate (7, 9) each have an attachment side (72, 92) for attaching the belts (70, 90) or side panels to a carrier web (8), and
wherein the two laminates are registered with one another before being cut such that the attachment side (92) of the second laminate (9) extends slightly in the lateral direction over the lateral edge of the first laminate (7); and
simultaneously cutting two individual belts or side panels from the registered laminates.

2. Method according to claim 1, wherein the first web portion and the second web portion are displaced with respect to one another such that a gap (14) is provided between them before the first elastic web material is joined, and joining the first elastic web material such that it bridges the gap and the cut line; or
wherein the first web portion and the second web portion are held in a close relationship with respect to one another, and the attachment positions of the first lateral side section and the second lateral side section on the first and second web portions are spaced apart from the cut line.

3. Method according to any one of the preceding claims, wherein the first and second elastic web materials are elastic in the cross machine direction (CD); and/or
wherein the first and second elastic web materials are essentially non-elastic in the machine direction (MD).

4. Method according to any one of the preceding claims, wherein wing tabs (6) for fastening the belts or side panels to a wearer are attached to at least one lateral side of the laminate at predetermined intervals.

5. Method according to claim 4, wherein the dimensions of the predetermined intervals correspond to the dimensions of the individual belts or side panels in the machine direction (MD) such that at least one wing tab is situated on each individual belt or the side panel.

6. Method according to any one of the preceding claims, wherein the first and second elastic web materials are each fed in the form of a single longitudinal strip of a dimension in the cross machine direction larger than the cross-machine dimension of the gap.

7. Method according to claim 6, wherein the cross-machine dimension of the first and second elastic web materials are smaller than the cross-machine dimension of the web of material before it is cut.

8. Method according to any one of the preceding claims, wherein the first and second elastic web materials (3, 4) are provided by cutting a single elastic web material along the machine direction (MD) into a first elastic web portion (34) and a second elastic web portion (36).

9. Method according to any one of the preceding claims, wherein the first web portion and the second web portion, on the one hand, and the third web portion and the fourth web portion, on the other hand, are spaced apart from one another by the same amount, resulting in an equally spaced gap between the first web of material and the second web of material.

10. Method according to any one of the preceding claims, wherein an absorbent structure (8), in particular including a back-sheet, a top-sheet and an absorbent core, is fed in the machine direction and the individual belts or the individual side panels are joined to the absorbent structure.

11. Method according to claim 10, wherein the individual belts or side panels are fixedly attached to the absorbent structure only on one lateral attachment side (72, 92) of that individual belt or side panel.

12. Method according to claim 11, wherein the other lateral side of that belt or side panel is attached to the absorbent structure by a releasable bond.

13. An absorbent article comprising at least two belts or side panels, said at least two belts or side panels being made in accordance with the method of claim 1.

## Patentansprüche

1. Verfahren zur Fertigung von mindestens zwei Gurten (70) oder Seitenflächen eines Absorptionsartikels, wobei das Verfahren die Schritte des Zuführens einer ersten Materialbahn (1) und einer zweiten Materialbahn (2) in der Maschinenrichtung (MD) umfasst;
Schneiden der ersten Materialbahn (1) entlang einer sich in Maschinenrichtung erstreckenden Schnittlinie in mindestens einen ersten Bahnabschnitt (10) und einen zweiten Bahnabschnitt (12), des Schneidens der zweiten Materialbahn (2) entlang der Maschinenrichtung und entlang einer Schnittlinie in mindestens einen dritten Bahnabschnitt (20) und einen vierten Bahnabschnitt (22);
Bereitstellen einer ersten elastischen Materialbahn und einer zweiten elastischen Materialbahn (3, 4) und Zuführen der elastischen Materialbahnen in der Maschinenrichtung;
Produzieren eines ersten Laminats (7)durch Befestigen des ersten elastischen Bahnmaterials an den ersten Bahnabschnitt und den zweiten Bahnabschnitt der ersten Materialbahn, um die Schnittlinie zu überbrücken, um im resultierenden Laminat Elastizität bereitzustellen;
Produzieren eines zweiten Laminats (9) durch Befestigen des zweiten elastischen Bahnmaterials an den dritten Bahnabschnitt und den vierten Bahnabschnitt des zweiten Bahnmaterials, um die Schnittlinie zu überbrücken, um im resultierenden Laminat Elastizität bereitzustellen; und
Schneiden von zwei einzelnen Gurten (70, 90) oder Seitenflächen aus den Laminaten entlang der Maschinenquerrichtung (CD), wobei das erste und zweite Laminat (7, 9) jeweils eine Befestigungsseite (72, 92) zum Befestigen der Gurte (70, 90) oder Seitenflächen an eine Trägerbahn (8) aufweisen, und
wobei die zwei Laminate vor dem Schneiden übereinandergelegt werden, sodass die Befestigungsseite (92) des zweiten Laminats (9) geringfügig in seitlicher Richtung über die Seitenkante des ersten Laminats (7) übersteht und
gleichzeitiges Schneiden von zwei Gurten oder Seitenflächen aus den übereinandergelegten Laminaten.

2. Verfahren nach Anspruch 1, wobei der erste Bahnabschnitt und der zweite Bahnabschnitt gegeneinander versetzt werden, sodass eine Lücke (14) zwischen ihnen bereitgestellt wird, bevor das erste elastische Bahnmaterial angefügt wird, und Verbinden des ersten elastischen Bahnmaterials, sodass es die Lücke und die Schnittlinie überbrückt; oder
wobei der erste Bahnabschnitt und der zweite Bahnabschnitt bezüglich einander in einer engen Beziehung gehalten werden und die Befestigungspositionen des ersten seitlichen Seitenabschnitts und des zweiten seitlichen Seitenabschnitts auf dem ersten und zweiten Bahnabschnitt von der Schnittlinie beabstandet werden.

3. Verfahren nach einem der vorstehenden Ansprüche, wobei das erste und das zweite elastische Bahnmaterial in der Maschinenquerrichtung (CD) elastisch sind; und/oder
wobei das erste und das zweite elastische Bahnmaterial in Maschinenrichtung (MD) im Wesentlichen unelastisch sind.

4. Verfahren nach einem der vorstehenden Ansprüche, wobei Flügellaschen (6) zum Befestigen der Gurte oder Seitenflächen an einen Träger an mindestens einer seitlichen Seite des Laminats in vorher festgelegten Abständen.

5. Verfahren nach Anspruch 4, wobei die Abmessungen der vorher festgelegten Abstände den Abmessungen der einzelnen Gurte oder Seitenflächen in der Maschinenrichtung (MD) entsprechen, sodass sich auf jedem einzelnen Gurt oder der Seitenfläche mindestens eine Flügellasche befindet.

6. Verfahren nach einem der vorstehenden Ansprüche, wobei das erste und zweite elastische Bahnmaterial in der Form eines einzelnen länglichen Streifens einer Abmessung in der Maschinenquerrichtung, größer als die Machinenquerabmessung der Lücke zugeführt werden.

7. Verfahren nach Anspruch 6, wobei die Maschinenquerabmessung des ersten und zweiten elastischen Bahnmaterials kleiner ist als die Maschinenquerabmessung des Bahnmaterials bevor es geschnitten wird.

8. Verfahren nach einem der vorstehenden Ansprüche, wobei das erste und zweite elastische Bahnmaterial (3, 4) durch Schneiden eines einzigen elastischen Bahnmaterials entlang der Maschinenrichtung (MD) in einen ersten elastischen Bahnabschnitt (34) und einen zweiten elastischen Bahnabschnitt (36) bereitgestellt werden.

9. Verfahren nach einem der vorstehenden Ansprüche, wobei der erste Bahnabschnitt und der zweite Bahnabschnitt einerseits, und der dritte Bahnabschnitt und der vierte Bahnabschnitt andererseits voneinander um die gleiche Menge beabstandet sind, was in einer gleichmäßig beabstandeten Lücke zwischen der ersten Materialbahn und der zweiten Material bahn resultiert.

10. Verfahren nach einem der vorstehenden Ansprüche, wobei eine absorbierende Struktur (8), insbesondere beinhaltend eine Unterschicht, eine Deckschicht und einen absorbierenden Kern, in der Maschinenrichtung zugeführt wird, und die einzelnen Gurte oder die einzelnen Seitenflächen an die absorbierenden Struktur angefügt werden.

11. Verfahren nach Anspruch 10, wobei die einzelnen Gurte oder Seitenflächen lediglich an der seitlichen Befestigungsseite (72, 92) des einzelnen Gurts oder Seitenfläche fest mit der absorbierenden Struktur verbunden werden.

12. Verfahren nach Anspruch 11, wobei die andere seitliche Seite des Gurts oder Seitenfläche durch eine lösbare Bindung mit der absorbierenden Struktur verbunden wird.

13. Absorptionsartikel, der mindestens zwei Gurte oder Seitenflächen umfasst, wobei die mindestens zwei Gurte oder Seitenflächen in Übereinstimmung mit dem Verfahren von Anspruch 1 gefertigt werden.

## Revendications

1. Procédé de fabrication d'au moins deux ceintures (70) ou panneaux latéraux d'un article absorbant, le procédé comprenant les étapes d'alimenter une première bande de matériau (1) et une deuxième bande de matériau (2) dans la direction de fabrication (MD);
découper la première bande de matériau (1) le long d'une ligne de découpe s'étendant le long de la direction de fabrication en au moins une première partie de bande (10) et une deuxième partie de bande (12), découper la deuxième bande de matériau (2) le long de la direction de fabrication et le long d'une ligne de découpe en au moins une troisième partie de bande (20) et une quatrième partie de bande (22);
fournir un premier matériau de bande élastique et un deuxième matériau de bande élastique et alimenter les matériaux de bande élastiques (3, 4) dans la direction de fabrication ;
produire un premier stratifié (7) en fixant le premier matériau de bande élastique à la première partie de bande et la deuxième partie de bande de la première bande de matériau de manière à enjamber la ligne de découpe afin de donner de l'élasticité au stratifié obtenu ;
produire un deuxième stratifié (9) en fixant le deuxième matériau de bande élastique à la troisième partie de bande et la quatrième partie de bande de la deuxième bande de matériau, de manière à enjamber la ligne de découpe afin de donner de l'élasticité au stratifié obtenu ; et
découper deux ceintures individuelles (70, 90) ou panneaux latéraux individuels à partir des stratifiés le long de la direction transversale (CD) à la direction de fabrication,
dans lequel les premier et deuxième stratifiés (7, 9) ont chacun un côté de fixation (72, 92) pour fixer les ceintures (70, 90) ou panneaux latéraux à une bande support (8), et
dans lequel les deux stratifiés sont alignés l'un avec l'autre avant d'être découpés de sorte que le côté de fixation (92) du deuxième stratifié (9) dépasse légèrement latéralement du bord latéral du premier stratifié (7) ; et
découper simultanément deux ceintures individuelles ou panneaux latéraux individuels à partir des stratifiés alignés.

2. Procédé selon la revendication 1, dans lequel la première partie de bande et la deuxième partie de bande sont décalées l'une par rapport à l'autre de telle sorte qu'un espace (14) est fourni entre elles avant que le premier matériau de bande élastique ne soit relié, et le premier matériau de bande élastique est relié de telle sorte qu'il enjambe l'espace et la ligne de découpe ; ou
dans lequel la première partie de bande et la deuxième partie de bande sont maintenues en relation étroite l'une par rapport à l'autre, et les positions de fixation de la première section latérale et de la deuxième section latérale sur les première et deuxième parties de bande sont espacées de la ligne de découpe.

3. Procédé selon l'une quelconque des revendications précédentes, dans lequel les premier et deuxième matériaux de bande élastiques sont élastiques dans la direction transversale (CD) à la direction de fabrication ; et/ou
dans lequel les premier et deuxième matériaux de bande élastiques sont essentiellement non élastiques dans la direction de fabrication (MD).

4. Procédé selon l'une quelconque des revendications précédentes, dans lequel des languettes latérales (6) pour attacher les ceintures ou panneaux latéraux à un porteur sont fixées à au moins un côté latéral du stratifié à des intervalles prédéterminés.

5. Procédé selon la revendication 4, dans lequel les dimensions des intervalles prédéterminés correspondent aux dimensions des ceintures individuelles ou panneaux latéraux individuels dans la direction de fabrication (MD) de sorte qu'au moins une languette latérale est située sur chaque ceinture individuelle ou le panneau latéral.

6. Procédé selon l'une quelconque des revendications précédentes, dans lequel les premier et deuxième matériaux de bande élastiques sont chacun alimentés sous la forme d'une bande longitudinale unique ayant une dimension dans la direction transversale à la direction de fabrication supérieure à la dimension transversale de l'espace.

7. Procédé selon la revendication 6, dans lequel la dimension transversale des premier et deuxième matériaux de bande élastiques est inférieure à la dimension transversale de la bande de matériau avant sa découpe.

8. Procédé selon l'une quelconque des revendications précédentes, dans lequel les premier et deuxième matériaux de bande élastiques (3, 4) sont obtenus en découpant un matériau de bande élastique unique le long de la direction de fabrication (MD) en une première partie de bande élastique (34) et une deuxième partie de bande élastique (36).

9. Procédé selon l'une quelconque des revendications précédentes, dans lequel la première partie de bande et la deuxième partie de bande, d'une part, et la troisième partie de bande et la quatrième partie de bande, d'autre part, sont espacées les unes des autres dans les mêmes proportions, créant ainsi un espace égal entre la première bande de matériau et la deuxième bande de matériau.

10. Procédé selon l'une quelconque des revendications précédentes, dans lequel une structure absorbante (8), en particulier une comprenant une feuille arrière, une feuille supérieure et une âme absorbante, est alimentée dans la direction de fabrication et les ceintures individuelles ou les panneaux latéraux individuels sont reliés à la structure absorbante.

11. Procédé selon la revendication 10, dans lequel les ceintures individuelles ou panneaux latéraux individuels sont fixés fermement à la structure absorbante uniquement sur un côté de fixation latéral (72, 92) de cette ceinture individuelle ou de ce panneau latéral individuel.

12. Procédé selon la revendication 11, dans lequel l'autre côté latéral de cette ceinture ou de ce panneau latéral est fixé à la structure absorbante par une liaison séparable.

13. Article absorbant comprenant au moins deux ceintures ou panneaux latéraux, ces aux moins deux ceintures ou panneaux latéraux étant fabriqués conformément au procédé de la revendication 1.
